# EUROPÄISCHE PATENTSCHRIFT

(11) **EP 0 369 937 B1**
(45) Veröffentlichungstag und Bekanntmachung des Hinweises auf die Patenterteilung: **01.09.1993**
(21) Anmeldenummer: 89730214.7
(22) Anmeldetag: 19.11.1989
(51) Int. Cl.: A61B 1/00

(54) **Endoskop, insbesondere Arthroskop**
Endoscope, in particular an arthroscope
Endoscope, en particulier un arthroscope

(30) Priorität: 18.11.1988 DE 8814573 U; 29.03.1989 DE 8904011 U
(43) Veröffentlichungstag der Anmeldung: 23.05.1990
(73) Patentinhaber: Effner Biomet GmbH, D-12247 Berlin (DE)
(72) Erfinder: Anapliotis, Emmanuel, D-1000 Berlin 33 (DE); Schich, Gisbert, D-8800 Ansbach (DE)
(74) Vertreter: Christiansen, Henning, Dipl.-Ing.

(56) Entgegenhaltungen:
- DE-A- 2 246 182
- DE-A- 3 727 190
- DE-B- 1 117 256
- DE-U- 7 833 379
- DE-U- 8 808 299
- FR-A- 1 293 091
- US-A- 3 297 022
- US-A- 4 756 304

## Beschreibung

Die Erfindung betrifft ein Endoskop der im Oberbegriff des Anspruchs 1 angegebenen Art.

Derartige Endoskope dienen insbesondere in Form von Arthroskopen der Untersuchung und Behandlung akuter Gelenkinnenräume, meist des Kniegelenks.

Es sind Endoskope bekannt, die eine gegenüber einer Geradeausblick-Optik zur optischen Achse geneigte Blickrichtung aufweisen. Der schräge Blickwinkel erlaubt es, durch Drehen des Instrumentes das durch die Einführungsrichtung festgelegte Blickfeld wesentlich zu erweitern.

Nachteilig bei einer Drehung des gesamten Instrumentes ist die Tatsache, daß Anschlüsse, Haltegriffe und Bildauswertesysteme, insbesondere der üblicherweise radial zur Längsachse des Instrumentes abstehende Anschlußstutzen für die Beleuchtung einschließlich des zu einer Lichtquelle führenden Lichtleitkabels mitgedreht werden müssen. Dadurch ist die Bewegungsfreiheit, insbesondere bei medizinischen Anwendungen, wesentlich beeinträchtigt. Insbesondere bei Operationsbedingungen stellt jede Zuleitung, welche das Gesichts- oder Arbeitsfeld einschränkt, eine bedeutsame Behinderung dar.

Aus der DE-A-3 727 190 ist ein rohrförmiges, am objektnahen Ende geschlossenes Führungsrohr für ein Endoskop bekannt, welches Lichtleiter aufweist und mit einer Lichtquelle gekoppelt ist. Nachdem dieses Führungsrohr in die zu untersuchende Körperpartie unter Bildung eines Hohlraums eingeführt worden ist und die angrenzenden zu untersuchenden Bereiche beleuchtet, wird ein Endoskop, welches nur eine zylinderförmige Optik aufweist, in das Führungsrohr eingeführt. Das Endoskop kann, da es keine Verbindung mit dem Führungsrohr eingeht, frei gedreht werden. Das Endoskop weist weiterhin eine gegenüber einer Geradeausblick-Optik zur optischen Achse geneigte Blickrichtung auf. Der schräge Blickwinkel erlaubt es, durch Drehen des Instrumentes das durch die Einführungsrichtung festgelegte Blickfeld wesentlich zu erweitern.

Nachteilig hierbei ist jedoch, daß eine eventuell mit der Optik verbundene Videokamera bei einer Verdrehung der Optik ebenfalls mitgedreht werden muß.

Bei einem weiteren aus der US-A-4 756 304 bekannten Endoskop ist dieses in bezug auf die angeschlossene Videokamera verdrehbar ausgebildet. Nachteilig bei diesem Endoskop ist, daß in diesem Fall bei einer Drehung des Endoskops der Anschlußstutzen für die Beleuchtung einschließlich des Lichtleiterkabels mitgedreht werden muß.

Der Erfindung liegt die Aufgabe zugrunde, die Handhabbarkeit und gleichzeitig die Einsatzmöglichkeiten eines Endoskops der eingangs genannten Gattung zu verbessern.

Diese Aufgabe wird mit den kennzeichnenden Merkmalen des Anspruchs 1 gelöst.

Die Erfindung beruht auf der Erkenntnis, daß eine zweiteilig ausgebildete Optik, deren Teile relativ zueinander verdrehbar sind, ein Verdrehen des objektnahen Abschnitts der Optik zur Änderung der Optikblickrichtung ermöglicht, ohne daß Kabelanschlüsse, Haltegriffe und/oder okularseitige Bildauswerte oder Aufzeichnungsgeräte mitgedreht werden.

Die optiktragende Fassung des erfindungsgemäßen Endoskops besteht dabei vorzugsweise aus zwei im wesentlichen zylinderförmigen Abschnitten, wobei die Trennstelle vom objektnahen Ende her gesehen hinter dem Beleuchtungsstutzen vorgesehen ist. Der objektnahe Teil des Endoskops besteht aus dem objektnahen Abschnitt der optiktragenden Fassung und einer diesen Abschnitt konzentrisch umschließenden lichtleiterführenden Hülse, wobei der objektnahe Fassungsabschnitt innerhalb der Hülse drehbar ist. Der Drehwinkel beträgt vorzugsweise bis zu 360°. Die lichtleiterführende Hülse hat relativ zum okularseitigen Abschnitt des Endoskopschaftes eine feste Lage. Drehbar ist nur der objektnahe Abschnitt der optiktragenden Fassung. Damit ist, wie bereits erwähnt, gewährleistet, daß zum Beispiel Bildauswerte- oder Bildaufzeichnungsgeräte, die am okularseitigen Abschnitt der optiktragenden Fassung angesetzt werden, ebenfalls eine feste Raumlage haben. Die Drehbarkeit des objektnahen Abschnittes der optiktragenden Fassung ermöglicht aufgrund der abgeknickten Blickrichtung der verwendeten Optik einen "Rundumblick".

Der verdrehbare Teil der Optikfassung ist bevorzugt bis zu einer der Handhabung zugänglichen Position in der Nähe des dem Benutzer zugewandten Endes geführt. Hier ist bevorzugt ein Bedienungselement angeordnet, das von außen ohne weiteres zugänglich ist, wenn die die Optik umgebenden Lichtleiter vorher seitlich abgezweigt sind. Hinter dem Bedienungselement ist dann eine "optische Schnittstelle" vorgesehen, welche den Übergang in einen feststehenden Teil des weiteren optischen Übertragungsweges ermöglicht. Hieran anschließend folgt der Anschluß für eine - ebenfalls feststehende - Videokamera.

Ein Haltegriff, der auch die abzweigende Zuleitung für den Lichtleiter und Anschlüsse für eventuelle Spülleitungen aufnimmt, "überbrückt" dabei mechanisch den in Form des Bedienungselementes von außen zugänglichen Teil der Optikfassung. Besonders vorteilhaft ist die Mehrfachfunktion eines derartigen Haltegriffes. Der Haltegriff dient neben der bequemen Halterung des Endoskops und der Kamera auch der Heranführung jeglicher Schläuche und Leitungen an das Endoskop. Behinderungen des Operateurs durch frei herabhängende Spül- und/oder Absaugschläuche sowie Lichtleitkabel werden dadurch vermieden.

Im Interesse einer ausreichenden Beleuchtung des Objektbereiches ist der Beleuchtungswinkel der Lichtleiter in ihrem Austrittsbereich an den gesamten möglichen Beobachtungsbereich angepaßt.

Die Lichtaustrittsflächen der Lichtleiter bilden bei Weitwinkelbeleuchtung vorzugsweise ein kegelstumpfartig oder konkav auslaufendes Ende des objektnahen Abschnittes. Vorteilhaft ist diese objektseitige Form insbesondere auch für arthroskopische Untersuchungen, da ein gleitender Übergang der Lichtaustrittsfläche zum Trokar geringere Knorpelverletzungen beim Einführen in das zu arthroskopierende Gelenk verursacht.

In einer speziellen Ausführungsform sind die Lichtleiter an ihren objektnahen Endungen in einen der Optikfassung unmittelbar benachbarten inneren Bereich mit kreisringförmiger Lichtaustrittsfläche und einen peripheren äußeren Bereich mit kegeliger Lichtaustrittsfläche geteilt. Die Teilung wird vorzugsweise durch das Einsetzen eines Ringes mit im wesentlichen dreieckigem Querschnitt realisiert.

Einer anderen Ausführungsform gemäß ist die gesamte Lichtaustrittsfläche kreisringförmig ausgebildet und mit einem mattierten Glasring verkleidet, wodurch eine weitwinklige Lichtstreuung erreicht wird. Der Glasring weist bevorzugt viertelkreisförmigem Querschnitt auf, wobei die gekrümmte Fläche einen die Verletzungsgefahr herabsetzenden stetigen Übergang von der Mantelfläche zur Stirnfläche des objektnahen Abschnitts bildet.

Die Trennstelle der Optikfassung kann beispielsweise als Ringschwalbe aber auch als Schraubverbindung mit Überwurfring ausgebildet sein. In beiden Fällen ist eine gute Zentrierung der beiden Abschnitte zueinander bei uneingeschränkter Drehbarkeit möglich. Um das ungewollte Abdrehen eines mit einem Gewinde versehenen Überwurfringes zu vermeiden, kann dieser durch Verkleben oder Arretierung mit einem Schaftabschnitt in seiner Position fixiert werden. Austauschbarkeit bzw. wahlweises Ansetzen verschiedener Schaftabschnitte ist dann allerdings nicht mehr möglich.

Zur bequemen Handhabung des drehbaren Teiles dient bevorzugt ein Rändelring, der an dem dem Anschlußstutzen für die Beleuchtung zugewandten Ende des objektnahen Abschnittes konzentrisch um dessen Längsachse angebracht ist.

Die Drehung läßt sich aber auch mit Hilfe eines batteriebetriebenen Motors erzeugen, wobei der Motor an dem beobachtungsseitigen Abschnitt, insbesondere an bzw. in einem Haltegriff befestigt ist und die Motorwelle direkt oder über Getriebeelemente an der Peripherie des objektnahen Abschnittes bzw. des Rändelringes angreift und diesen in rotierende Bewegung versetzt.

Die Trennstelle ermöglicht das Verdrehen des objektnahen relativ zum objektfernen Abschnitt der Optikfassung, kann aber gegebenenfalls auch dem wahlweisen Anschluß verschiedener objektnaher bzw. objektferner Abschnitte dienen.

Weitere vorteilhafte Ausführungen des erfindungsgemäßen Endoskops betreffen Varianten zur Anpassung der Beleuchtungsrichtung an die Blickrichtung.

Vorzugsweise werden dazu mit der Spitze der Optikfassung verbundene Umlenkprismen in die Beleuchtungshülse eingeschoben und durch Federwirkung in den Beleuchtungsstrahlengang ausgeklappt. Beim Drehen der Optikfassung innerhalb der Beleuchtungshülse werden die Prismen mitgedreht, so daß die Beleuchtungsrichtung für jede Drehstellung mit der Blickrichtung übereinstimmt.

Eine weitere Variante zur Beeinflussung der Beleuchtungsrichtung beruht auf der Verformung eines die Lichtaustrittsfläche der Beleuchtungshülse abdeckenden, elastischen und lichtdurchlässigen Ringes durch Berührung nach innen überstehender Bereiche dieses Ringes beim Einschieben und beim Drehen der Optikfassung.

Entsprechend einer weiteren Variante ist eine auf der Lichtaustrittsfläche der Beleuchtungshülse drehbar gelagerte Abdeckung aus formfestem transparentem Material, insbesondere Glas, vorgesehen, deren Form der eines Ablenkprismas mit zentrischer Bohrung für den Durchtritt des Beobachtungsstrahlenganges entspricht. Durch die Abdeckung wird das Licht um einen festen Winkel abgelenkt. Eine Optikfassung mit einer Schrägblickoptik passenden Blickwinkels ist mit der Abdeckung arretierbar, so daß die Abdekkung zusammen mit der Optikfassung verdrehbar ist und das Licht in die jeweilige Blickrichtung umlenkt.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 einen Längsschnitt durch ein Arthroskop mit einer Trennstelle,
Figur 2 eine andere Ausführungsform eines Arthroskops gemäß Figur 1,
Figur 3 das ausgeleuchtete und das beobachtete Objektfeld zu den Figuren 1 und 2,
Figur 4 eine Ringschwalbenausführung einer Trennstelle,
Figur 5a einen Schnitt durch einen Seitenbereich des objektnahen Endes einer ersten Ausführungsform einer Weitwinkelbeleuchtung,
Figur 5b einen Schnitt durch einen Seitenbereich des objektnahen Endes einer zweiten Ausführungsform einer Weitwinkelbeleuchtung,
Figur 6 eine perspektivische Ansicht eines Endoskops gemäß Figur 1,
Figuren 6a bis 6e Einzelteile des Endoskopes gemäß Figur 6,
Figur 7 eine erste Variante einer motorischen Rändelringverstellung,
Figur 8 eine zweite Variante einer motorischen Rändelringverstellung.
Figur 9a eine erste Ausführungsvariante des distalen Endes eines Endoskops während der Einsetzphase einer Optikfassung mit Schrägblickoptik in teilweise geschnittener Darstellung,
Figur 9b die Ansicht gemäß Figur 9a nach dem Einsetzen der Optikfassung,
Figur 9c eine Stirnansicht zu Figur 9b,
Figur 9d ein Prisma in perspektivischer Darstellung als Detail der Figuren 9a, 9b bzw. 9c, die
Figuren 10a, 10b, 10c und 10d eine zweite Ausführungsvariante in der Darstellungsweise der Figuren 9a, 9b, 9c und 9d, die
Figuren 11a, 11b, und 11c eine dritte Ausführungsvariante in der Darstellungsweise der Figuren 9a, 9b und 9c und die
Figuren 12a, 12b und 12c eine vierte Ausführungsvariante in der Darstellungsweise der Figuren 9a, 9b und 9c.

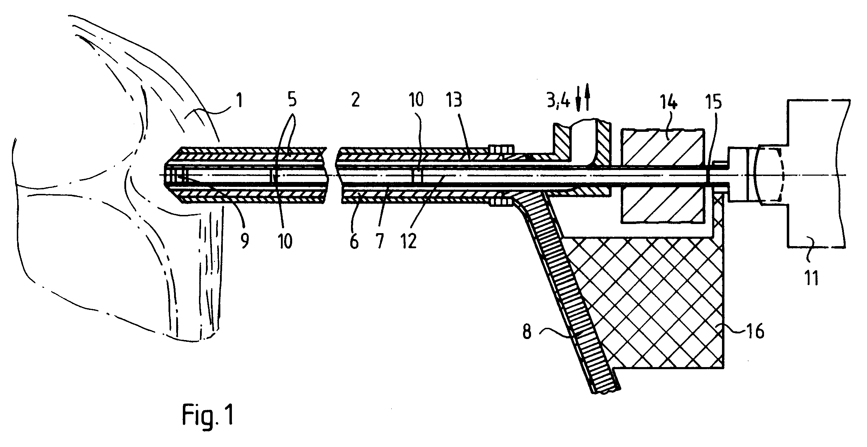

In Figur 1 ist ein erfindungsgemäßes Endoskops in der Ausführung als Arthroskop zur Untersuchung des Kniegelenk-Innenraumes 1 im Längsschnitt dargestellt. Ein derartiges Arthroskop besteht aus einer Trokarhülse 2, einem Saug- 3 und/oder Spülkanalanschluß 4 und einer endoskopischen Einrichtung 5, die in die Trokarhülse 2 einschiebbar ist. Die endoskopische Einrichtung 5 setzt sich aus Bauteilen für die Beleuchtung und die Objektbeobachtung zusammen. Dabei sind für die Beleuchtung üblicherweise Lichtleitfasern 6 vorgesehen, welche konzentrisch um eine Optikfassung 7 angeordnet sind und über einen seitwärts abstehenden Beleuchtungsstutzen 8 sowie einem Lichtleitkabel mit einer Lichtquelle verbunden sind. Zwischen den Lichtleitfasern 6 und der Optikfassung 7 verläuft der Saug- und/oder Spülkanal. Zur Stabilisierung und zur Abstandshaltung der Lichtleitfasern 6 gegenüber der Optikfassung 7 sind in der Figur nicht dargestellte Stege vorgesehen. Die Optikfassung 7 enthält ein Objektiv 9 sowie eine Anzahl von Bildversetzungslinsen 10, die das Objekt 1 zur visuellen Beobachtung in die Zwischenbildebene eines Okulars oder zur Monitorbeobachtung bzw. Bildaufzeichnung auf das Endikon einer Kamera 11 abbilden. Die optische Achse 12 des Endoskops 5 fällt zusammen mit der geometrischen Längsachse des Trokars 2.

Im Folgenden ist mit "Optikfassung" nur der drehbare, objektnahe Abschnitt der optiktragenden Fassung gemeint.

Die Optikfassung 7 ist relativ zu einer feststehenden Kamera 11 koaxial verdrehbar, wohingegen der aus den Lichtleitfasern 6 bestehende Beleuchtungsteil 13 feststeht.

Hinter dem Beleuchtungsstutzen 8 ist ein von außen zugänglicher Rändelring 14 vorgesehen, der seinerseits mit der Optikfassung 7 fest verbunden ist. Eine Trennstelle 15 hinter dem Rändelring 14 gestattet ein Verdrehen der Optikfassung 7 gegenüber dem Kamerateil 11.

Auf diese Weise wird erreicht, daß der Beleuchtungsanschlußstutzen 8, der Saug- 3 und/oder Spülkanalanschluß 4, die Kamera 11 sowie ein Griff bzw. eine Halterung 16 bei Drehung der Optikfassung 7 eine konstante Raumlage beibehalten und sich nicht mitdrehen.

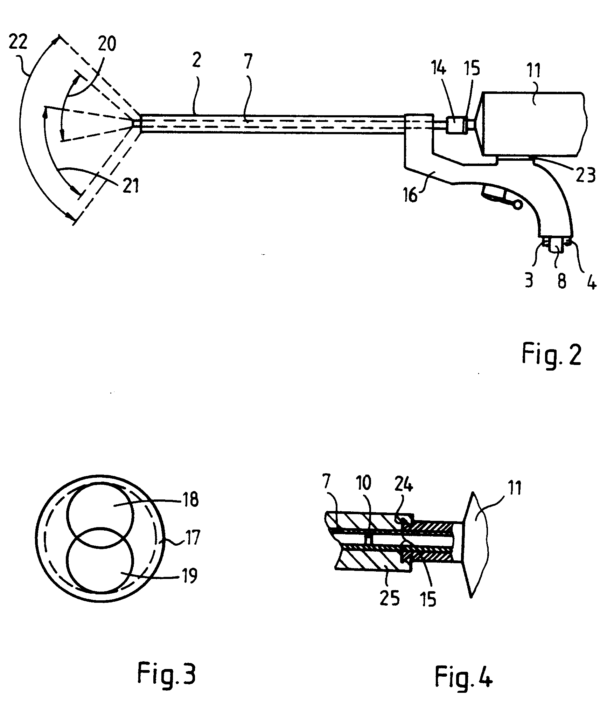

Figur 2 zeigt eine weitere prinzipielle Darstellung des zuvor beschriebenen Ausführungsbeispiels.

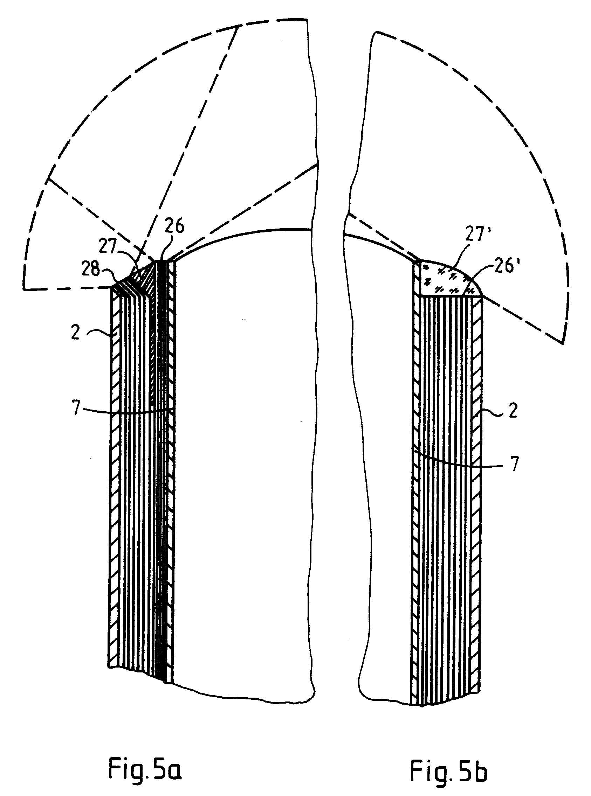

In Figur 2 und Figur 3 sind die optischen Winkelverhältnisse für zwei um 180° zueinander gedrehte Positionen der Optikfassung 7 dargestellt. Bei zur optischen Achse 12 geneigter Blickrichtung wird ein Feld 17 (Figur 3) ausgeleuchtet, das wesentlich größer als das Sehfeld 18 bzw. 19 (Figur 3) ist. In einer bestimmten Drehstellung wird der Winkelbereich 20 und damit das Objektfeld 18 anvisiert und in einer um 180° zu dieser Stellung gedrehten Position der Winkelbereich 21 und damit das Objektfeld 19. Der beleuchtete Raumwinkel 22 bzw. das ausgeleuchtete Feld 17 ist konstant. Vorteilhafte Ausführungsformen des objektseitigen Lichtaustrittsendes für eine solcherart weitwinklige Ausleuchtung sind in den Figuren 5a und 5b dargestellt.

Der Griff 16 bildet bei dem in Figur 2 wiedergegebenen Ausführungsbeispiel eine brückenartige Verbindung zwischen der Trokarhülse 2 und der feststehenden Kamera 11 und ist gleichzeitig Träger für die vor dem Rändelring 14 abzweigenden Anschlüsse und Leitungen, insbesondere dem Beleuchtungsanschlußstutzen 8 sowie dem Saug- bzw. Spülkanalanschluß 3, 4. Die Kamera 11 ist mittels eines Aufsteckschuhs 23 jederzeit von dem Griff 16 abtrennbar, so daß die Optikfassung herausziehbar ist.

Figur 4 zeigt eine spezielle Variante einer Trennstelle 15 in Form einer Ringschwalbenverbindung 24. Diese Art der Verbindung von zylindrischen Teilen zeichnet sich durch exakte Justierbarkeit, einfache Bauweise und universelle Anwendbarkeit aus. Der zum objektseitigen Ende gewandte Teil 25 der Ringschwalbenverbindung 24 kann auch als Betätigungsmittel für die Drehbewegung dienen und ist dann zweckmäßigerweise als Rändelring ausgeführt.

Figur 5a zeigt eine erste Variante einer Weitwinkelbeleuchtung. Diese ist durch zwei voneinander getrennte ringförmige Lichtaustrittsflächen gekennzeichnet. Eine innere, der Optikfassung 7 unmittelbar benachbarte kreisringförmige Lichtaustrittsfläche 26 ist durch einen Ring 27 mit im wesentlichen dreieckigem Querschnitt von einer kegeligen Lichtaustrittsfläche 28 getrennt. Die beiden Lichtaustrittsbereiche 26 und 28 und der als Abstandshalter fungierende Ring 27 bilden eine glatte Fläche. Diese glatte Fläche wird bei Vorhandensein einer Trokarhülse 2 weiter fortgesetzt. Gewebeschädigungen beim Einführen des Endoskops bzw. Arthroskops werden dadurch weitgehend vermieden. Die Winkelbereiche des Lichtaustritts der beiden Lichtaustrittsbereiche überlappen sich wie in Figur 5a gestrichelt dargestellt. Es ist auch ersichtlich, daß auf diese Weise eine sehr weitwinklige Beleuchtung erzielt wird.

Bei einer zweiten, in Figur 5b dargestellten Variante ist die gesamte Lichtaustrittsfläche 26' kreisringförmig ausgebildet. Statt eines Abstandsringes 27 ist hier ein mattierter Glasring 27' vorgesehen, der ebenflächig auf der Lichtaustrittsfläche 26' aufliegt und annähernd viertelkreisförmigen Querschnitt aufweist. Die Mattierung bewirkt eine weitwinklige Streuung des Lichts und durch die abgerundete Querschnittsform des Glasringes 27' werden scharfkantige, verletzungsgefährdende Ecken vermieden.

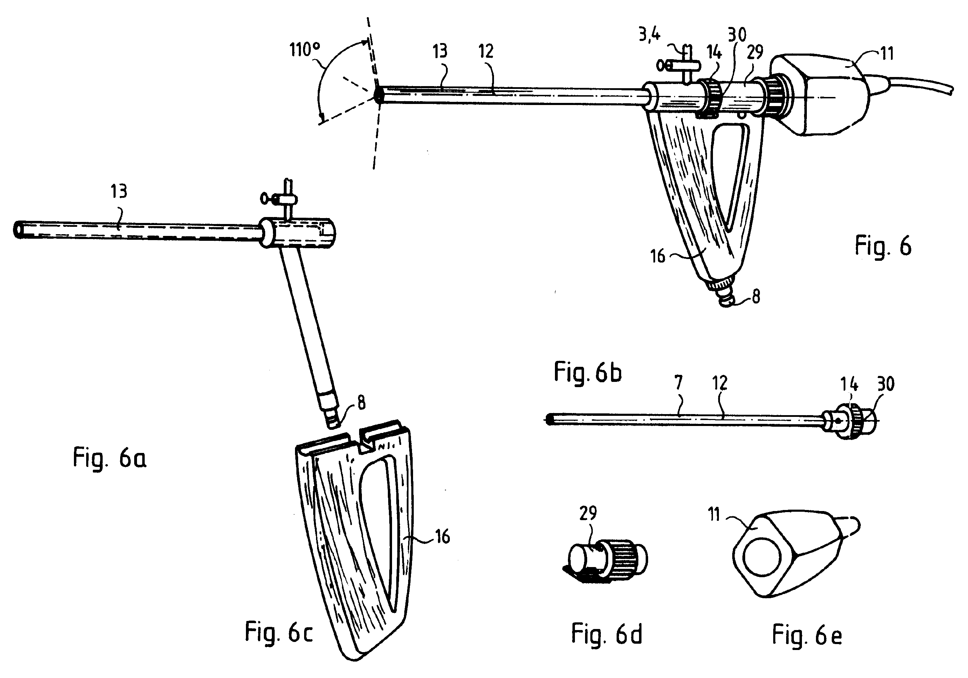

Figur 6 zeigt eine perspektivische Ansicht eines Arthroskopes gemäß dem Prinzip des in Figur 1 veranschaulichten Ausführungsbeispiels. Die Einzelteile und die Montagereihenfolge sind in den Figuren 6a bis 6e dargestellt. Das Endoskop besteht im wesentlichen aus einer Lichtleitfaserbeleuchtungseinrichtung 13 mit Beleuchtungsstutzen 8 (Figur 6a), einer stabförmigen Optikfassung 7 mit einem Rändelring 14 (Figur 6b) zur Drehung der Optikfassung 7 um ihre optische Achse 12, einem Anschluß 3 oder/und 4 für einen zwischen der Optikfassung 7 und der Beleuchtungseinrichtung 13 sich erstreckenden Saug oder/und Spülkanal, einem Griff 16 (Figur 6c) und einem Adapter 29 (Figur 6d) zur Anpassung an eine Kamera 11 (Figur 6e). Der Beleuchtungsstutzen 8 ist vorteilhaft in den Griff 16 integriert.

Bei Betätigung des Rändelringes 14 wird die Optikfassung 7 innerhalb des Saug/Spülkanales und der Beleuchtungsbaugruppe 13 um ihre optische Achse 12 gedreht. Der Rändelring 14 befindet sich in unmittelbarer Nähe des Griffes 16 und ist daher leicht zugänglich. Die momentane Blickrichtung ist mittels einer Markierung 30 am Rändelring 14 erkennbar. Vorteilhafterweise stimmt die radiale Lage der Rändelringmarkierung 30 mit der radialen Sehrichtung überein. Es ist auch möglich, eine Markierung zur Anzeige der Stellung des Rändelringes 14 und damit der Blickrichtung in die optische Abbildung einzuspiegeln.

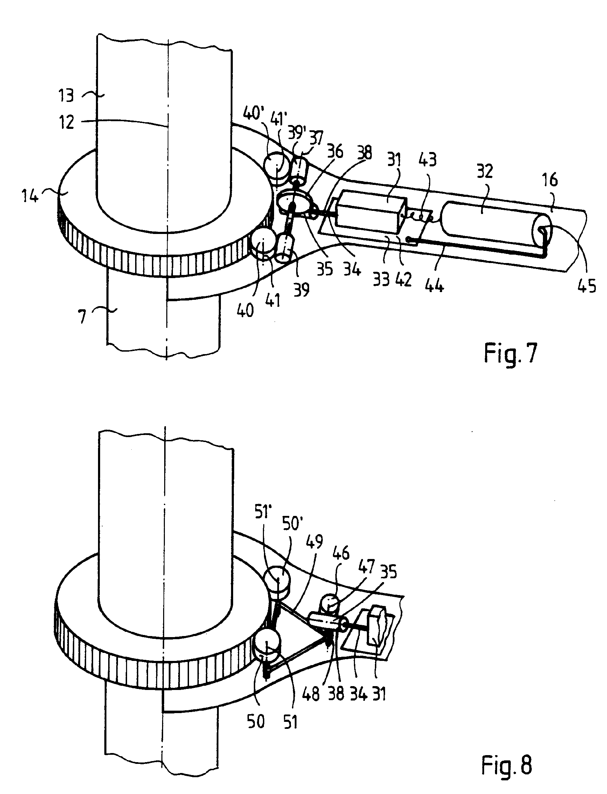

Die in den Figuren 7 und 8 dargestellten Varianten motorischer Verstelleinrichtungen für den Rändelring 14 bestehen aus einem im Griff 16 des Endoskopes bzw. Arthroskopes eingebauten Motor 31, einer ebenfalls im Griff 16 angeordneten Batterie 32, einem Schiebeschalter 33 und diversen Getriebeelementen, die die Energie des Motors 31 auf den Rändelring 14 übertragen und so eine Drehbewegung der Optikfassung 7 gegenüber der Beleuchtungsbaugruppe 13 bewirken.

Die Getriebeelemente sind bei der ersten, in Figur 7 dargestellten Variante als Schneckenrad/Ritzel-Kombinationen ausgebildet. Die Motorwelle 34 trägt ein Schneckenrad 35, welches sich mit einem Ritzel 36 in Eingriff befindet. Die Drehachse 37 des Ritzels 36 ist in einer radialen Ebene des Rändelringes 14 und senkrecht zur Drehachse 38 des Schneckenrades 35 angeordnet. Das Ritzel 36 ist über seine Achse starr mit zwei Schneckenrädern 39 und 39' verbunden. Diese Schneckenräder 39 und 39' greifen in zwei Ritzel 40 und 40' ein, deren Drehachsen 41 und 41' senkrecht zur radialen Ebene des Rändelringes 14 und senkrecht sowie symmetrisch zur Drehachse 38 des Schneckenrades 35 gerichtet sind. Diese Ritzel 40 und 40' sind gleichzeitig die Antriebselemente für den Rändelring 14. Die Art der Rändelung entspricht praktischerweise etwa einer zahnstangenartigen Ausformung.

Zum Ein- und Ausschalten des Motors 31 dient ein Schiebeschalter 33, der sich am Griff 16 des Endoskopes bzw. Arthroskopes an ergonomisch günstiger Stelle befindet. Bei Betätigung des Schiebeschalters 33 werden nicht nur der Stromkreis unterbrochen oder geschlossen, sondern gleichzeitig der Kraftschluß zwischen den Antriebselementen und dem Rändelring 14 unterbrochen oder hergestellt. Diese Doppelfunktion ist günstig, weil damit eine manuelle Drehbarkeit des Rändelringes 14 möglich bleibt. Zur Realisierung dieser Doppelfunktion ist der Schiebeschalter 33 mit einer verschieblichen Grundplatte 42 verbunden, auf der die Getriebeelemente, der Motor 31 sowie eine elastische Leitungsverbindung 43 und eine Verbindungsleitung 44 mit einem schaltbaren Kontakt 45 zwischen dem Motor 31 und der Batterie 32 montiert sind. Bei Betätigung des Schiebeschalters 33 in Richtung zum Rändelring 14 wird die Grundplatte 42 mitverschoben, wobei der Kontakt 45 mit einem Pol der Batterie 32 verbunden und gleichzeitig die Antriebselemente an die Peripherie des Rändelringes 14 angedrückt werden. Der Kontakt 43 zwischen dem auf der Grundplatte 42 verschieblichen Motor 31 und der fest in dem Gehäuse des Griffes 16 eingesetzten Batterie 32 bleibt aufgrund der elastischen Eigenschaften des Kontaktes 43 unabhängig vom Schaltzustand ständig bestehen.

Ein weiteres bevorzugtes Ausführungsbeispiel, insbesondere in Bezug auf die Getriebeelemente, ist in Figur 8 dargestellt. Die Motorwelle 34 trägt analog dem Ausführungsbeispiel gemäß Figur 7 ein Schneckenrad 35. In diesem greift ein Ritzel 46 ein, dessen Drehachse 47 sowohl senkrecht zur Drehachse 38 des Schneckenrades 35 als auch senkrecht zu einer radialen Rändelringebene steht. Auf der Achse des Schneckenrades 46 sitzt ein Reibrad 48, das einen Riemen 49 antreibt, welcher wiederum zwei Rollen 50 und 50' antreibt. Die Drehachsen 51 und 51' der beiden Rollen 50 und 50' stehen wie die des Ritzels 46 und damit des Reibrades 48 senkrecht zur Drehachse 38 des Schneckenrades 35 und senkrecht zur Rändelringebene. Die Rollen 50 und 50' sind außerdem spiegelsymmetrisch zur Ebene der Drehachsen 38 und 47 derart angeordnet, daß sie die Peripherie des Rändelringes 14 berühren. Bei entsprechenden Oberflächeneigenschaften der Peripherie des Rändelringes 14 und der Rollen 50 und 50' versetzen diese den Rändelring 14 durch Reibschluß in drehende Bewegung.

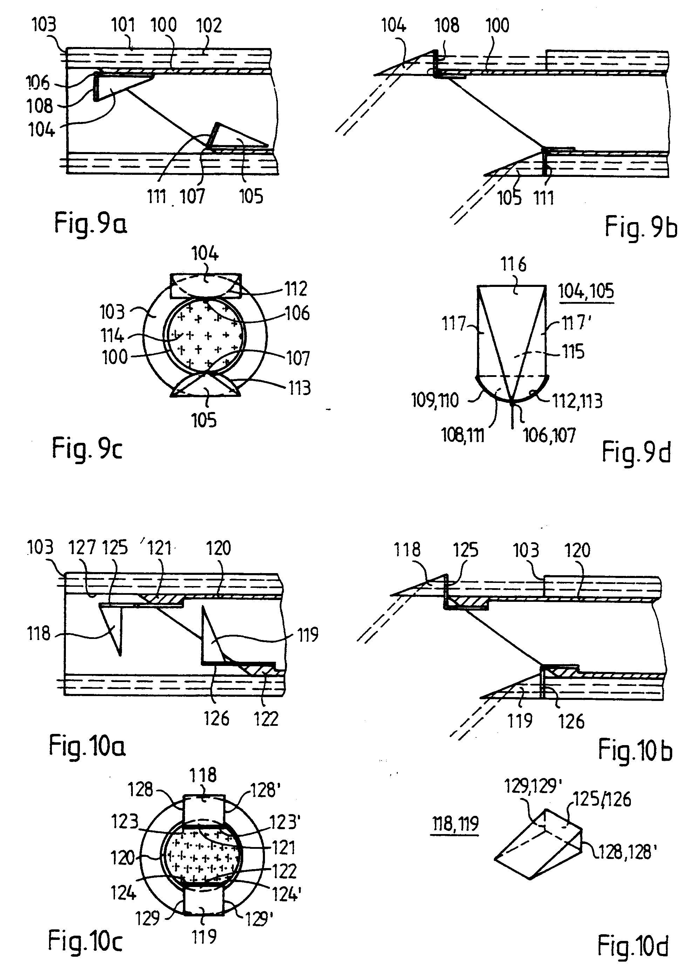

Die Figuren 9a und 9b zeigen das objektseitige Ende eines erfindungsgemäßen Endoskops während und nach dem Einsetzen einer Optikfassung 100 mit Schrägblickoptik in eine hülsenförmige Beleuchtungseinrichtung 101 mit Geradeausbeleuchtung. Die der Beleuchtung dienenden Lichtleitfasern 102 enden an einer kreisringförmigen objektseitigen Lichtaustrittsfläche 103. Zur Anpassung der Beleuchtungsrichtung an die Schrägblickoptik ist die in die Beleuchtungshülse 101 einzusetzende Optikfassung 100 mit zwei Winkelprismen 104 und 105 versehen, deren Ablenkungswinkel dem Blickwinkel der Schrägblickoptik angepaßt sind. Die Prismen 104 und 105 sind mit Scharnieren 106 und 107 am äußeren objektseitigen Rand der Optikfassung 100 befestigt, wobei sie sich gegenüberliegen. Die betreffenden, mit den Scharnieren 106 und 107 verbundenen Randbereiche der Optikfassung 100 sind dem am weitesten in die Beleuchtungshülse 101 hineinragenden sowie dem am weitesten zurückliegenden Bereich des Winkelobjektivs zugeordnet. Sobald die Optikfassung 100 so weit in die Beleuchtungshülse 101 hineingeschoben ist, daß ein Prisma 104 über den Beleuchtungsrand hinüberragt, klappt dieses Prisma 104 um 180° nach außen um, wodurch seine Grundfläche 108 zu der Lichtaustrittsfläche 103 der Lichtleitfaserbeleuchtungseinrichtung parallel ausgerichtet ist. Der Umklappvorgang wird durch eine in Figur 9d dargestellte Feder 109 ausgelöst, die das Bestreben hat, das Prisma 104 in die in der Figur 9b dargestellte Anordnung umzuklappen. Ein Anschlag, der bewirkt, daß das Prisma 104 nicht über 180° hinaus umklappt, ist in das Scharnier 106 eingebaut.

Das Scharnier 107 weist keinen derartigen Anschlag auf, da das zugehörige Prisma 105 durch die Wirkung einer in der Figur 9d dargestellten Feder 110 mit seiner Grundfläche 111 bis zur Lichtaustrittsfläche 103 umklappt und somit die gewünschte Lichtablenkung bewirkt. Zwischen dem anderen Prisma 104 und der Lichtaustrittsfläche 103 ist hingegen bei vollständig eingesetztem Optikschaft 100 ein Luftabstand vorhanden.

Beim Herausziehen der Optikfassung 100 aus der Beleuchtungshülse 101 klappen die beiden Prismen 104 und 105 wieder zurück und schmiegen sich mit einer entsprechend der Innenkrümmung der Optikfassung 100 abgerundeten Kante 112 bzw. 113 an die Innenwandung der Optikfassung 100. Ein in den Figuren 9a und 9b nicht dargestelltes Winkelobjektiv ist unterhalb des Klappbereiches der Prismen 104 und 105 in die Optikfassung 100 eingesetzt. Die dadurch hervorgerufene Abschattung des Sehfeldes ist jedoch im allgemeinen unbedeutend, zumal die Prismen mit zunehmendem Winkel der Schrägblickoptik immer kürzer werden und demzufolge weniger Raum zum Umklappen benötigen.

Figur 9c zeigt eine Frontansicht der Endoskopspitze, wobei die Lichtaustrittsfläche 103, die Prismen 104 und 105 in ausgeklapptem Zustand, die Stirnfläche der Optikfassung 100 und das Winkelobjektiv 114 erkennbar sind.

Der Aufbau der beiden Prismen 104 und 105 einschließlich der Scharniere 106 und 107 sowie der Federn 109 und 110 ist in Figur 9d noch einmal perspektivisch wiedergegeben. Das Prisma 104 bzw. 105 wird von der Grundfläche 108 bzw. 111, einer Reflexionsfläche 115, einer Lichtaustrittsfläche 116 und zwei abgerundeten Seitenflächen 117 und 117' begrenzt. Die Seitenflächen 117 und 117' bilden in der Projektion rechtwinklige Dreiecke.

In den Figuren 10a, 10b, 10c und 10d ist eine zweite Ausführungsvariante einer mit Prismen 118 und 119 versehenen Optikfassung 120 in gleichartiger Darstellungsweise wie die Figuren 9a, 9b, 9c und 9d wiedergegeben. Im Unterschied zu der ersten Variante werden die Prismen 118 und 119 nur um 90° umgeklappt. Die Optikfassung 120 ist an ihrem objektseitigen Ende mit zwei nach innen gerichteten und sich gegenüberliegenden Ansätzen 121 und 122 versehen. Durch die Ansätze 121 und 122 erhält die rohrförmige Optikfassung zwei ebene Bereiche, an denen die Prismen 118 und 119 mittels jeweils zweier Scharniere 123, 123' und 124, 124' befestigt sind. Während des Einsetzvorganges der Optikfassung 120 werden die rechteckigen Grundflächen 125 und 126 der Prismen 118 und 119 - wie Figur 10a zeigt - unmittelbar oberhalb der Ansätze 121 und 122 an der rohrförmigen Innenwandung 127 der Beleuchtungshülse entlanggeschoben, wobei lediglich die Seitenkanten 128, 128' und 129, 129' der Grundflächen 125 und 126 diese Innenwandung 127 berühren. Im eingeschobenen, in Figur 10b veranschaulichten Zustand sind die Prismen 118 und 119 um 90° nach außen in den Beleuchtungsstrahlengang umgeklappt.

Eine Frontansicht mit den beiden umgeklappten Prismen 118 und 119, der Lichtaustrittsfläche, der Optikfassung 120 und dem Winkelobjektiv ist in Figur 10c dargestellt.

Figur 10d zeigt eine perspektivische Ansicht des Prismas 118 bzw. 119. Durch die Rechteckform der Grundfläche 125 bzw. 126 ist dieses Prisma 118 bzw. 119 einfacher als das in Figur 9d dargestellte Prisma 104 bzw. 105 der ersten Variante aufgebaut.

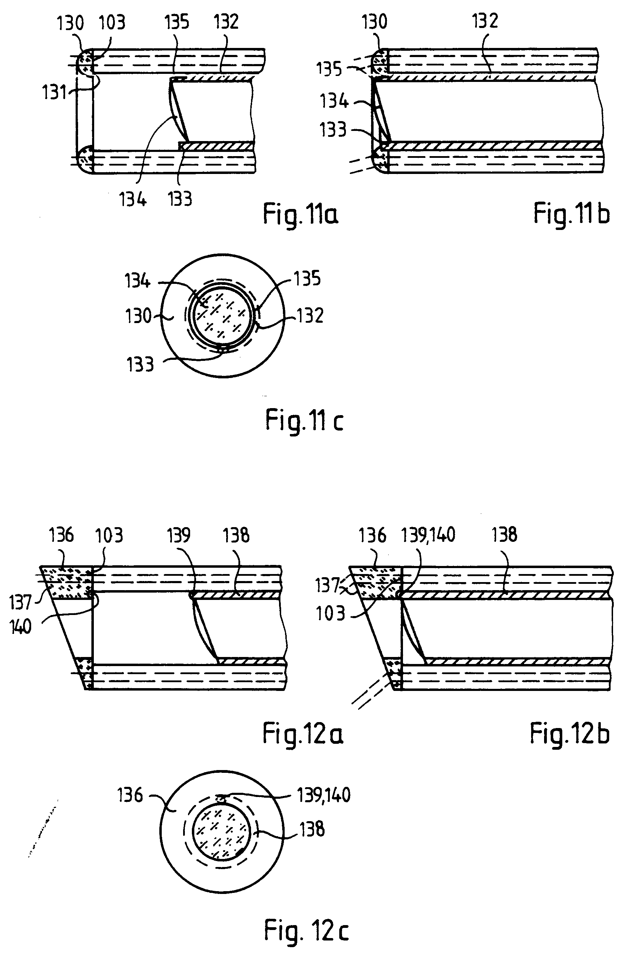

Bei der in den Figuren 11a, 11b und 11c dargestellten dritten Ausführungsvariante einer Endoskopspitze ist die gesamte Lichtaustrittsfläche 103 mit einem Ring 130 aus elastischem, durchsichtigem Material verkleidet, wobei der Querschnitt des Ringes 130 etwa halbkreisförmig ausgebildet ist. Der Ring 130 weist einen Bereich 131 auf, der geringfügig über die innere Begrenzung der Lichtaustrittsfläche 103 hinüberragt. Dieser Bereich 131 wird durch das Einschieben der Optikfassung 132 angestoßen, wodurch sich der gesamte Ring 130 derartig verformt, daß die Beleuchtungsrichtung weitgehend mit der Blickrichtung der Schrägblickoptik übereinstimmt. Die Optikfassung 132 ist dazu mit einem nach distal gerichteten Vorsprung 133 versehen, welcher der tiefsten Stelle des Winkelobjektivs 134 zugeordnet ist. Das objektseitige Ende der Optikfassung 132 weist an dem verbleibenden Umfangsbereich eine Wandstärkeverringerung 135 auf, so daß der vorstehende Bereich 131 des Ringes 130 nur von dem Vorsprung 133 berührt werden kann. Wird die Optikfassung 132 innerhalb der Beleuchtungshülse zum Zwecke eines "Rundumblickes" gedreht, berührt der mit der Optikfassung 132 verbundene Vorsprung 133 den Ring 130 auf einer kreisförmigen Bahn, wodurch immer andere Teile des Ringes 130 massiv elastisch verformt werden. Um ein Zurückfedern der Optikfassung 132 an dem elastischen Ring 130 zu vermeiden, können Rastungen zwischen der Optikfassung und der Beleuchtungshülse vorgesehen sein.

Auch bei der in den Figuren 12a, 12b und 12c wiedergegebenen Ausführungsvariante ist die Lichtaustrittsfläche 103 mit einer geringfügig nach innen überstehenden, ringförmigen Abdeckung 136 aus durchsichtigem Material verkleidet. Diese Abdeckung 136 ist jedoch im Gegensatz zu dem Ring 130 nicht elastisch ausgebildet. Die Abdeckung 136 liegt vollflächig auf der Lichtaustrittsfläche 103 der Lichtleitfaserbeleuchtungseinrichtung auf und ist zu dieser drehbar gelagert. Die der Auflagefläche gegenüberliegende Lichtaustrittsfläche 137 der Abdeckung 136 ist zur Auflagefläche bzw. Lichtaustrittsfläche 103 der Lichtleitfaserbeleuchtungseinrichtung geneigt, so daß das Licht in eine Richtung abgelenkt wird. Damit die Ablenkungsrichtung des Lichts mit der Blickrichtung der Optik übereinstimmt, ist eine einfache Arretierung der Optikfassung 138 und der Abdeckung 136 vorgesehen. Dazu ist der am weitesten nach distal gerichtete Stirnflächenbereich der Optikfassung 138 mit einem Verriegelungsnoppen 139 versehen, der in eine entsprechende Aussparung 140 an dem nach innen überstehenden Bereich der Auflagefläche der Abdeckung 136 eingreift, wobei die Aussparung 140 dem am weitesten nach distal vorstehenden Abdeckungsbereich zugeordnet ist. Bei Drehung der Optikfassung 138 wird die Abdeckung mitgedreht, wodurch Blickrichtung der Schrägblickoptik und Beleuchtungsrichtung in jeder Drehstellung übereinstimmen.

## Patentansprüche

1. Endoskop, insbesondere Arthroskop, bestehend aus einem optik- und einem lichtleitertragenden (7 und 6), im wesentlichen zylinderförmigen Schaft (2), wobei die optische Achse mit der Längsachse des Schaftes (2) übereinstimmt und die Lichtleiter (6) vorzugsweise konzentrisch um die Optik (7) herum angeordnet sind, sowie einem vorzugsweise radial zur Längsachse des Schaftes (2) gerichteten Beleuchtungsanschlußstutzen (8), über den die Lichtleiter (6) mit einem zu einer Lichtquelle führenden Lichtleitkabel verbunden sind, wobei
die Lichtleiter (6) in bezug auf den Beleuchtungsanschlußstutzen (8) räumlich fest angeordnet sind,
die Blickrichtung am objektnahen Ende der Optik (7) zur Längsachse geneigt ist und
die Optik (7) in bezug auf den Beleuchtungsanschlußstutzen (8) verdrehbar ausgebildet ist,
**dadurch gekennzeichnet,**
daß die Optik (7) mittels einer Trennstelle (15) in einen objektnahen und einen objektfernen Abschnitt unterteilt ist, welche relativ zueinander verdrehbar angeordnet sind, und
daß ein mit dem objektfernen Abschnitt der Optik (7) verbundener Haltegriff (16) vorgesehen ist, der den Beleuchtungsanschlußstutzen (8) trägt, der - vom objektnahen Ende des Endoskops her gesehen - vor der Trennstelle (15) gelegen ist.

2. Endoskop nach Anspruch 1, **dadurch gekennzeichnet,** daß der Drehwinkel der in bezug auf den Beleuchtungsanschlußstutzen (8) verdrehbaren Optik (7) im wesentlichen 360° beträgt.

3. Endoskop nach Anspruch 1, **dadurch gekennzeichnet,** daß an der Trennstelle (15) eine Ringschwalbe (24) vorgesehen ist.

4. Endoskop nach Anspruch 1, **dadurch gekennzeichnet,** daß an der Trennstelle (15) eine Schraubverbindung mit Überwurfring vorgesehen ist.

5. Endoskop nach Anspruch 4, **dadurch gekennzeichnet,** daß Mittel zum Festlegen der Verschraubung des Überwurfringes, insbesondere Klebemittel oder Arretiermittel vorgesehen sind.

6. Endoskop nach einem der Ansprüche 3 bis 5, **dadurch gekennzeichnet**, daß ein Rändelring (14) an einem der Trennstelle (15) benachbarten Bereich des drehbaren objektnahen Abschnitts der Optik (7) konzentrisch um dessen Längsachse (12) angeordnet ist.

7. Endoskop nach einem der vorangehenden Ansprüche, **dadurch gekennzeichnet,** daß ein batteriebetriebener Motor (31) vorgesehen ist, der mit dem objektfernen Abschnitt der Optik (7) verbunden ist und dessen Welle (34) direkt oder über Getriebeelemente an der Peripherie des in bezug auf den Beleuchtungsanschlußstutzen (8) drehbaren objektnahen Abschnitts der Optik (7) angreift und diesen in rotierende Bewegung versetzt.

8. Endoskop nach einem der vorangehenden Ansprüche, **dadurch gekennzeichnet**, daß der Haltegriff (16) einen Saug- und/oder Spülkanal (3, 4) aufnimmt.

9. Endoskop nach einem der vorangehenden Ansprüche, **dadurch gekennzeichnet**, daß der Öffnungswinkel des Beleuchtungskegels (22) größer als der objektseitige Öffnungswinkel (20 bzw. 21) des Beobachtungsstrahlenganges ist.

10. Endoskop nach einem der vorangehenden Ansprüche, **dadurch gekennzeichnet,** daß die Lichtleiter (6) derart ausgebildet sind, daß der Öffnungswinkel (22) des Beleuchtungskegels mindestens 110° beträgt.

11. Endoskop nach einem der vorangehenden Ansprüche, **dadurch gekennzeichnet,** daß die objektseitige Lichtaustrittsfläche der Lichtleiter (6) im wesentlichen die Form eines Teils einer Kegelstumpfoberfläche oder die Form einer entsprechenden Oberfläche eines konkav gekrümmten Rotationskörpers aufweist.

12. Endoskop nach einem der vorangehenden Ansprüche, **dadurch gekennzeichnet,** daß die Lichtleiter (6) an ihren objektnahen Endungen einen der Optik (7) unmittelbar benachbarten inneren Bereich mit kreisringförmiger Lichtaustrittsfläche (26) und einen peripheren äußeren Bereich mit kegeliger Lichtaustrittsfläche (28) bilden, wobei ein Ring (27) mit im wesentlichen dreieckigem Querschnitt zur Teilung der beiden Bereiche vorgesehen ist.

13. Endoskop nach einem der Ansprüche 1 bis 11, **dadurch gekennzeichnet,** daß ein eine kreisringförmig ausgebildete Lichtaustrittsfläche (26') abdeckender Ring (27') aus mattiertem Glas vorgesehen ist.

14. Endoskop nach Anspruch 13, **dadurch gekennzeichnet,** daß der Ring (27') viertelkreisförmigen Querschnitt aufweist, wobei die gekrümmte Fläche eine Außenfläche des objektseitigen Endes bildet.

15. Endoskop nach einem der vorangehenden Ansprüche, **dadurch** **gekennzeichnet,** daß am objektnahem Ende der Optikfassung (100, 120) mindestens ein um eine Kante klappbares Umlenkprisma (104, 105 bzw. 118, 119) vorgesehen ist, das während des Einsetzens der Optikfassung (100 bzw. 120) in eine Beleuchtungshülse (101) sowie während des Entnahmevorganges in die Optikfassung (100 bzw. 120) eingeklappt ist und im eingesetzten Zustand der Optikfassung (100 bzw. 120) mit Hilfe mindestens einer Feder (109, 110) derart nach außen umklappt, daß seine Lichteintrittsfläche (108, 111 bzw. 125, 126) zu der Lichtaustrittsfläche (103) der Beleuchtungshülse (101) parallel ausgerichtet ist, wobei die Umlenkrichtung und der Umlenkwinkel der Blickrichtung und dem Blickwinkel der Optik (114) entsprechen.

16. Endoskop nach Anspruch 15, **dadurch gekennzeichnet,** daß das Umlenkprisma (104, 105) um 180° umklappbar ist und eine gekrümmte Kante zwischen der Lichteintrittsfläche (108, 111) und einer gekrümmten Lichtaustrittsfläche (115) aufweist, wobei die Kante mit einem Klappscharnier (106, 107) versehen ist und die Krümmung der Kante bzw. der Lichtaustrittsfläche (115) der Innenkrümmung der Optikfassung (100) entspricht.

17. Endoskop nach Anspruch 15, **dadurch gekennzeichnet,** daß das Umlenkprisma (118, 119) um 90° umklappbar ist und eine gerade Kante zwischen der Lichteintrittsfläche (125, 126) und einer Lichtaustrittsfläche aufweist, wobei die Kante mit mindestens einem Klappscharnier (123 und 123', 124 und 124') versehen ist und innerhalb des kreisförmigen Querschnitts der Optikfassung (120) sehnenartig eingebettet ist.

18. Endoskop nach einem der Ansprüche 1 bis 14, **dadurch gekennzeichnet,** daß die Lichtaustrittsfläche (103) der Beleuchtungshülse mit einem Ring (130) aus elastischem transparentem Material sowie halbkreisförmigem Querschnitt und einem die Lichtaustrittsfläche (103) geringfügig nach innen überragenden Bereich (131) versehen ist und daß die Optikfassung (132) einen Vorsprung (133) aufweist, der den überstehenden Bereich (131) des Ringes (130) bei Berührung derartig verformt, daß das Licht entsprechend der Blickrichtung und dem Blickwinkel der Optik abgelenkt wird.

19. Endoskop nach einem der Ansprüche 1 bis 14, **dadurch gekennzeichnet,** daß eine auf der kreisringförmigen Lichtaustrittsfläche (103) der Beleuchtungshülse drehbar gelagerte Abdeckung (136) aus formfestem transparentem Material vorgesehen ist, deren Lichtaustrittsfläche (137) zur Lichtaustrittsfläche (103) der Beleuchtungshülse geneigt ist und daß die Optikfassung (138) mit der Abdeckung (136) derartig arretierbar ist, daß das Licht entsprechend der Blickrichtung und dem Blickwinkel der Optik abgelenkt wird.

## Claims

1. An endoscope, more particularly an arthroscope, comprising a substantially cylindrical shaft (2) carrying the optics and light conductors (7 and 6), the optical axis corresponding to the longitudinal axis of the shaft (2) and the light conductors (6) being, preferably, arranged concentrically about the optics (7), a light connecting piece (8), preferably oriented radially relative to the longitudinal axis of the shaft (2), via which light connecting piece the light conductors (6) are connected to a light-conducting cable leading to a light source,
the light conductors (6) being spatially fixedly arranged relative to the light connecting piece (8),
the line of vision at the end nearer the object of the optics (7) being at an angle, relative to the longitudinal axis, and
the optics (7) being constructed to be rotatable, relative to the lighting connecting piece (8), characterised in that the optics (7) are sub-divided into a section nearer the object and a section more remote from the object, by means of a disconnecting point (15), said sections being arranged so as to be rotatable relative to one another, and in that a handle (16) connected to the section more remote from the object of the optics (7) is provided which carries the light connecting piece (8) positioned in front of the disconnecting point (15), when viewed from the end of the endoscope nearer the object.

2. An endoscope according to claim 1, characterised in that the angle of rotation of the optics (7) rotatable relative to the light connecting piece (8) is substantially 360°.

3. An endoscope according to claim 1, characterised in that a dovetailed ring (24) is provided at the disconnecting point (15).

4. An endoscope according to claim 1, characterised in that a screw connection with a screw collar ring is provided at the disconnecting point (15).

5. An endoscope according to claim 4, characterised in that means are provided for securing the screw coupling of the screw collar ring, more particularly adhesive or locking means.

6. An endoscope according to any one of claims 3 to 5, characterised in that a knurled ring (14) is arranged in a region of the rotatable section nearer the object of the optics (7) so as to be concentric about the longitudinal axis (12) thereof, said region being close to the connecting point (15).

7. An endoscope according to any one of the preceding claims, characterised in that a battery-operated motor (31) is provided which is connected to the section more remote from the object of the optics (7) and whose shaft (34) engages either directly or via gear members on the periphery of the section nearer the object of the optics (7), rotatable relative to the light conducting piece (8), and sets said section into a rotating motion.

8. An endoscope according to any one of the preceding claims, characterised in that the handle (16) receives a suction and/or rinsing-channel (3, 4).

9. An endoscope according to any one of the preceding claims, characterised in that the aperture angle of the illumination cone (22) is greater than the aperture angle on the object side (20 or 21) of the observation light path.

10. An endoscope according to any one of the preceding claims, characterised in that the light conductors (6) are formed such that the aperture angle (22) of the illumination cone is at least 110°.

11. An endoscope according to any one of the preceding claims, characterised in that the light exit surface on the object side of the light conductors (6) is substantially in the form of a partial surface of a frustum or a corresponding surface of a concavely curved rotational member.

12. An endoscope according to any one of the preceding claims, characterised in that the light conductors (6) form, at the ends nearer the object, an inner region directly adjacent to the optics (7), which has a circular light exit (26) and a peripheral outer region with a cone-like light exit surface (28), a ring (27) with a substantially triangular cross-section being provided for separating the two regions.

13. An endoscope according to any one of claims 1 to 11, characterised in that a ring (27') made of frosted glass is provided which covers a circularly formed light exit surface (26').

14. An endoscope according to claim 13, characterised in that the ring (27') has a quadrant cross-section, the curved surface forming an outer face of the end on the object side.

15. An endoscope according to any one of the preceding claims, characterised in that at least one deviating prism (104, 105 or 118, or 119) which may be tilted by means of one edge is provided at the end nearer the object, said deviating prism being retracted in a light sleeve (101) during the insertion of the optics mount (100 or 120) and in the optics mount (100 or 120) during the removal process and, when the optics mount (100 or 120) is in the state of insertion, being folded over by means of at least one spring (109, 110) so that its light entry surface (108, 111 or 125, 126) is in parallel alignment to the light exit surface (103) of the lighting sleeve (101), the deviating direction and the deviating angle corresponding to the direction and angle of view of the optics (114).

16. An endoscope according to claim 15, characterised in that the deviating prism (104, 105) may be folded over through 180° and has a curved edge between the light entry surface (108, 111) and a curved light exit surface (115), the edge being provided with a folding hinge (106, 107) and the curvature of the edge or of the light exit surface (115) corresponding to the inner curvature of the optics mount (100).

17. An endoscope according to claim 15, characterised in that the deviating prism (118, 119) may be folded over through 90° and has a straight edge between the light entry surface (125, 126) and a light exit surface, the edge being provided with at least one folding hinge (123 and 123', 124 and 124') and being embedded sinewlike inside the circular cross-section of the optics mount.

18. An endoscope according to any one of claims 1 to 14, characterised in that the light exit surface (103) of the lighting sleeve has a ring (130) made of resilient transparent material as well as a semi-circular cross-section and a region (131) slightly inwardly projecting beyond the light exit surface (103), and in that the optics mount (132) has a projection (133) which deforms the protruding region (131) of the ring (130) upon contact, such that the light is deflected to correspond to the direction and angle of view of the optics.

19. An endoscope according to any one of claims 1 to 14, characterised in that a cover (136) made of dimensionally stable transparent material is provided, which is rotatably mounted on the circular light exit surface (103) of the lighting sleeve and whose light exit surface (137) is inclined, relative to the light exit surface (103) of the lighting sleeve, and in that the optics mount (138) may be locked with the cover (136) so that the light is deflected to correspond to the direction and angle of view of the optics.

## Revendications

1. Endoscope, en particulier arthroscope, constitué d'une canule (2) de forme sensiblement cylindrique et portant l'optique et des conducteurs de la lumière (7 et 6), l'axe optique coincidant avec l'axe longitudinal de la canule (2) et les conducteurs de la lumière (6) étant de préférence disposés concentriquement autour de l'optique (7), ainsi que d'une tubulure (8) de raccord d'éclairement qui est de préférence orientée radialement par rapport à l'axe longitudinal de la canule (2) et par l'intermédiaire de laquelle les conducteurs de lumière (6) sont reliés à un câble conducteur de la lumière qui conduit à une source de lumière, endoscope dans le cas duquel
les conducteurs de la lumière (6) sont disposés fixes dans l'espace par rapport à la tubulure (8) de raccord d'éclairement,
la direction d'observation, à l'extrémité de l'optique (7) proche de l'objet, est inclinée par rapport à l'axe longitudinal et
l'optique (7) est conçue avec liberté de rotation par rapport à la tubulure (8) de raccord d'éclairement,
endoscope caractérisé
par le fait que l'optique (7) est, au moyen d'une position de sectionnement (15), divisée en une portion proche de l'objet et une portion éloignée de l'objet, portions qui sont disposés avec liberté de rotation l'une par rapport à l'autre,
par le fait qu'est prévue une poignée (16) qui est reliée à la portion de l'optique (7) éloignée de l'objet et qui porte la tubulure (10) de raccord d'éclairement qui est située en avant de la position de sectionnement (15) - vu de l'extrémité de l'endoscope proche de l'objet.

2. Endoscope selon la revendication 1, caractérisé par le fait que l'angle de rotation de l'optique (7) qui peut tourner par rapport à la tubulure (8) de raccord d'éclairement vaut sensiblement 360°.

3. Endoscope selon la revendication 1, caractérisé par le fait qu'une queue d'aronde annulaire (24) est prévue à la position de sectionnement (15).

4. Endoscope selon la revendication 1, caractérisé par le fait qu'à la position de sectionnement (15) est prévue une liaison par vissage avec bague-chapeau.

5. Endoscope selon la revendication 4, caractérisée par le fait que sont prévus des moyens de fixation du vissage de la bague-chapeau, en particulier des moyens de collage ou des moyens de blocage.

6. Endoscope selon l'une des revendications 3 à 5, caractérisé par le fait qu'une bague moletée (14) est disposée en une zone, voisine de la position de sectionnement (15), de la portion de l'optique (7) proche de l'objet et pouvant tourner, disposition concentrique autour de l'axe longitudinal (12) de cette portion de l'optique.

7. Endoscope selon l'une des revendications précédentes, caractérisé par le fait qu'est prévu un moteur (31) qui est entrainé par une batterie, qui est relié avec la portion de l'optique (7) éloignée de l'objet et dont l'arbre (34) attaque, directement ou par l'intermédiaire d'éléments mécaniques, la périphérie de la portion de l'optique (7), proche de l'objet, qui peut tourner par rapport à la tubulure (8) du raccord d'éclairement et entraine en un mouvement de rotation cette portion de l'optique.

8. Endoscope selon l'une des revendications précédentes, caractérisé par le fait que la poignée (16) reçoit un canal d'aspiration et/ou de lavage (3, 4).

9. Endoscope selon l'une des revendications précédentes, caractérisé par le fait que l'angle d'ouverture du cône d'éclairement (22) est supérieur à l'angle d'ouverture (20 ou 21), côté objet, du chemin du faisceau d'observation.

10. Endoscope selon l'une des revendications précédentes, caractérisé par le fait que les conducteurs de la lumière (6) sont conçus de façon que l'angle d'ouverture (22) du cône d'éclairement vaut au moins 110°.

11. Endoscope selon l'une des revendications précédentes, caractérisé par le fait que la surface de sortie de la lumière, côté objet, des conducteurs de la lumière (6) présente sensiblement la forme d'une partie d'une surface de tronc de cône ou la forme d'une surface correspondante d'un corps de révolution de courbure concave.

12. Endoscope selon l'une des revendications précédentes, caractérisé par le fait que les conducteurs de la lumière (6) forment, à leurs extrémités proches de l'objet, une zone intérieure, immédiatement voisine de l'optique (7), avec surface de sortie de la lumière (26) en forme de bague circulaire, ainsi qu'une zone périphérique extérieure avec surface la lumière conique (28), une bague (27) de section sensiblement triangulaire étant prévue pour séparer ces deux zones.

13. Endoscope selon l'une des revendications 1 à 11, caractérisée par le fait qu'est prévue une bague (27'), en verre dépoli, qui recouvre une surface de sortie de la lumière (26') en forme de bague circulaire.

14. Endoscope selon la revendication 13, caractérisée par le fait que la bague (27') présente la forme d'un quart de cercle, la surface incurvée formant une surface extérieure de l'extrémité côté objet.

15. Endoscope selon l'une des revendications précédentes, caractérisé par le fait qu'à l'extrémité de la monture optique (100, 120) proche de l'objet est prévu au moins un prisme (104, 105 ou 118, 119) de déviation de la lumière qui peut se rabattre autour d'une arête, qui, pendant l'insertion de la monture optique (100 ou 120) dans une canule d'éclairement (101) ainsi que pendant le processus d'extraction, est rabattu vers l'intérieur dans la monture optique (100 ou 120) et qui, lorsque la monture optique (100 ou 120) est à l'état inséré, se rabat vers l'extérieur, à l'aide d'au moins un ressort (109, 110) de façon telle que sa surface de sortie de la lumière (108, 111 ou 125, 126) s'oriente parallèlement à la surface de sortie de la lumière (103) de la canule d'éclairement (101), la direction de déviation de la lumière et l'angle de déviation correspondant à la direction d'observation et à l'angle d'observation de l'optique (114).

16. Endoscope selon la revendication 15, caractérisé par le fait que le prisme (104, 105) de déviation de la lumière peut se rabattre de 180° et présente, entre la surface d'entrée de la lumière (108, 111) et une surface incurvée (115) de sortie de la lumière, une arête incurvée, étant précisé que cette arête présente une charnière de rabattement (106, 107) et que la courbure de l'arête ou celle de la surface de sortie de la lumière (115) correspond à la courbure intérieure de la monture optique (100).

17. Endoscope selon la revendication 15, caractérisé par le fait que le prisme (118, 119) de déviation de la lumière peut se rabattre de 90° et présente, entre la surface d'entrée de la lumière (125, 126) et une surface de sortie de la lumière, une arête rectiligne, cette arête présentant au moins une charnière de rabattement (123 et 123', 124 et 124'), et s'insérant, à la façon d'une corde, à l'intérieur de la section de forme circulaire de la monture optique (120).

18. Endoscope selon l'une des revendications 1 à 14, caractérisé par le fait que la surface de sortie de la lumière (103) de la canule d'éclairement comporte une bague (130 en un matériau élastique transparent et de section en forme de demi-cercle, ainsi qu'une zone (131) qui déborde légèrement vers l'intérieur au-delà de la surface de sortie de la lumière (103° et par le fait que la monture optique (132) présente une saillie (103) qui, lors du contact, déforme la zone débordante (131) de la bague (130) de façon telle que la lumière est déviée conformément à la direction d'observation et à l'angle d'observation de l'optique.

19. Endoscope selon l'une des revendications 1 à 14, caractérisé par le fait qu'est prévu un chaperon (36), en matériau transparent de forme fixe, qui est porté, avec liberté de rotation, sur la surface de sortie de la lumière (103), en forme de bague circulaire, de la canule d'éclairement et dont la surface de sortie de la lumière (137) est inclinée par rapport à la surface de sortie de la lumière (103) de la canule d'éclairement, et par le fait que la monture optique (138) peut être bloquée avec le chaperon (136) de façon telle que la lumière soit déviée conformément à la direction d'observation et à l'angle d'observation de l'optique.
